# EUROPÄISCHE PATENTSCHRIFT

(11) **EP 0 516 786 B1**
(45) Veröffentlichungstag und Bekanntmachung des Hinweises auf die Patenterteilung: **24.05.1995**
(21) Anmeldenummer: 92901309.2
(22) Anmeldetag: 14.12.1991
(51) Int. Cl.: D02G 1/02, G01N 33/36

(54) **VERFAHREN ZUR REGELUNG DER FADENZUGKRAFT**
METHOD OF CONTROLLING YARN TENSION
PROCEDE POUR LE REGLAGE DE LA FORCE DE TENSION DE FIL

(30) Priorität: 19.12.1990 DE 4040587; 19.01.1991 DE 4101502
(43) Veröffentlichungstag der Anmeldung: 09.12.1992
(73) Patentinhaber: BARMAG AG, D-42862 Remscheid (DE)
(72) Erfinder: LORENZ, Hellmut, D-5630 Remscheid (DE); NEUMANN, Bernd, D-5608 Radevormwald (DE)
(74) Vertreter: Pfingsten, Dieter, Dipl.-Ing.
(86) Internationale Anmeldenummer: DE9100981
(87) Internationale Veröffentlichungsnummer: WO9211535

(56) Entgegenhaltungen:
- EP-A- 0 271 252
- EP-A- 0 289 009
- DE-A- 3 306 594

## Beschreibung

Die Erfindung betrifft ein Verfahren zur Regelung der Fadenzugkraft nach dem Oberbegriff des Anspruchs 1.

Dieses Verfahren ist durch die DE-A-33 06 594 bekannt. Dabei wird das von dem Friktionsfalschdraller auf den Faden übertragene Drallmoment in Abhängigkeit von der Zugkraft verstellt, die hinter dem Friktionsfalschdraller gemessen wird. Diese Verstellung des Drallmomentes erfolgt in dem gezeigten Beispiel dadurch, daß die Anpreßkraft der auf den Faden einwirkenden Oberflächen verstellt wird. Mit diesem Verfahren kann die Fadenzugkraft auf einen konstanten Wert eingeregelt werden. Diese Regelung hat den Vorteil, daß der Durchmesser und die Dichte der hergestellten Aufwickelspulen vorgegeben und für viele Spulstellen gleichmäßig eingestellt und gleichzeitig die Standzeit der verwendeten Reibelemente ohne Qualitätseinbuße erhöht werden kann.

Es sei darauf hingewiesen, daß in der Beschreibung der hier vorliegenden Erfindung und des Standes der Technik die Begriffe "Fadenzugkraft" und "Fadenspannung" als gleichbedeutend verwendet wurden, obwohl "Fadenspannung" an sich als die spezifische, d.h. als auf die Fadenfeinheit bezogene Fadenzugkraft definiert ist.
Es wurde jedoch bei diesem Verfahren der Nachteil beobachtet, daß die Regelung der Fadenspannung hinter dem Friktionsaggregat, die zwar als wesentliches Kriterium für die Qualität des Texturierergebnisses gilt, gleichwohl mit Schwankungen des Texturierergebnisses einhergeht.

Dieser Nachteil wird vermieden durch die Lösung nach Anspruch 1.

Es erfolgt also eine Regelung der Fadenspannung T2, indem ein Langzeitwert LW der Fadenspannung mit einem Sollwert verglichen und die Reibkraft in Abhängigkeit von der Differenz gesteuert wird. Der Regelbereich wird jedoch in einen Bereich verlegt, der durch diese Erfindung definiert ist. Es wird hierdurch ermöglicht, die Fadenparameter, die für die Qualität des Fadens von ausschlaggebender Bedeutung sind, einzeln und unabhängig voneinander zu überwachen und einzustellen sowie zu optimieren. Dadurch kann das Endprodukt, nämlich Faden wie auch die erzeugte Spule sehr wesentlich verbessert werden. Zusätzlich ist es möglich, eine Qualitätsüberwachung durchzuführen. Hierzu wird auf die EP-A-0 439 183 Stand der Technik Art. 54(3) EPÜ ausdrücklich verwiesen. Danach wird bevorzugt das Verstellsignal, durch welches die Fadenspannung ausgeregelt wird, daraufhin überwacht, ob es den vorgegebenen Bereich zwischen dem oberen Grenzwert und dem unteren Grenzwert verläßt. Diese oberen bzw. unteren Grenzwerte werden auch im Rahmen dieser Erfindung zum Zwecke der Ausgabe eines Qualitätssignals oder zur Ausgabe eines Alarmsignals benutzt, wenn das Verstellsignal den Bereich zwischen diesen Grenzwerten verläßt. Zusätzlich kann aber auch, wie dies ebenfalls in der genannten EP-A-0 439 183 vorgesehen ist, die Differenz zwischen der aktuell gemessenen Fadenspannung T2 nach einer geeigneten Umformung mit dem Verstellsignal verglichen und ein Alarmsignal oder ein Qualitätssignal ausgegeben werden, wenn das Differenzsignal einen vorgegebenen Bereich zwischen einem oberen Grenzwert und einem unteren Grenzwert des Differenzsignales verläßt.

Zur Beeinflussung der Drallübertragung steht insbesondere das Mittel zur Verfügung, die Drehgeschwindigkeit des Friktionsfalschdrallers zu beeinflussen. Eine Beeinflussung der Fadenzugkraft hinter dem Drallgeber ist jedoch auch über eine geometrische Verstellung am Drallgeber möglich, z.B. durch gezielte Veränderung des Achsabstandes.

Bei einem Friktionsfalschdraller, der aus zwei Oberflächen besteht, die den Faden zwischen sich einklemmen, vgl. z.B. EP-A-22 743 A1, US-A-4,145,871 oder US-A-4,248,038, wird vorzugsweise der Andruck verstellt. Ein Friktionsfalschdraller, der aus drei in den Ecken eines gleichschenkeligen Dreiecks angeordneten Achsen mit darauf aufgespannten, sich im Zentrum des Dreiecks überlappenden Scheiben besteht, zeichnet sich zur Ausführung des Verfahrens insbesondere dadurch aus, daß der Achsabstand, der die Scheiben aufspannenden Achsen verstellt werden kann. Die Verstellung der Achsen kann insbesondere dadurch geschehen, daß zwei Achsen auf Exzentern gelagert werden, welche durch eine Antriebseinrichtung verstellt werden können.

Im folgenden werden Ausführungsbeispiele der Erfindung anhand der Zeichnung beschrieben.

Es zeigt:
- Fig. 1: eine Bearbeitungsstelle einer Falschzwirnkräuselmaschine in schematischer Darstellung;
- Fig. 2: ein Einstelldiagramm für die Regelung der Fadenzugkraft in der Drallzone.

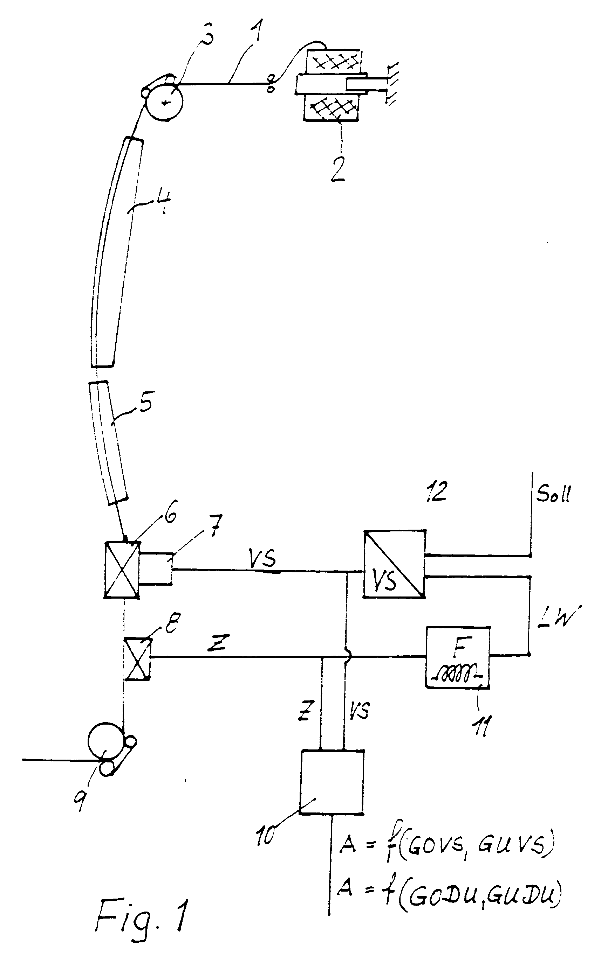

Die Figur 1 zeigt eine schematische Darstellung einer Bearbeitungsstelle einer Falschzwirnkräuselmaschine. Der synthetische Faden 1 wird durch das Eingangslieferwerk 3 von der Vorlagespule 2 abgezogen. Die Texturierzone wird zwischen dem Eingangslieferwerk 3 und dem Abzugslieferwerk 9 gebildet. Sie umfaßt vor allem eine Heizschiene 4, eine Kühlschiene 5 und den Friktionsfalschdraller 6. Der Friktionsfalschdraller weist endlos bewegte Oberflächen auf, die quer zur Fadenachse bewegt sind und an denen der Faden anliegt. Diese Oberflächen erteilen dem Faden in Richtung Eingangslieferwerk eine Zwirnung, die sich in Richtung des Ausgangslieferwerks 9 wieder auflöst.

Zwischen dem Friktionsfalschdraller 6 und dem Ausgangslieferwerk 9 ist ein Fadenzugkraftmeßinstrument 8 angeordnet, durch welches die Fadenzugkraft, auch "Fadenspannung" genannt, gemessen und als Ausgangssignal weitergegeben wird. Es sei bemerkt, daß hinter dem Ausgangslieferwerk 9 eine Aufwicklung oder noch eine Zwischenbehandlung durch Erwärmung folgt.

Das Ausgangssignal Z des Fadenzugkraftmessers 8, welches die gemessene Fadenzugkraft repräsentiert, wird über einen Filter 11 in einen Langzeitwert LW umgeformt. Der Langzeitwert LW wird gemeinsam mit einem Sollwert einer Regeleinrichtung 12 zugeführt. In der Regeleinrichtung 12 werden der Sollwert und der Langzeitwert miteinander verglichen und in eine Verstellgröße VS umgeformt. Durch die Verstellgröße VS wird ein Stellglied 7 verstellt, durch welches die Drallübertragung des Friktionsfalschdrallers 6 auf den Faden 1 gesteuert wird.

Das Ausgangssignal Z des Fadenzugkraftmessers 8 wird ebenso wie das Verstellsignal VS einer Auswerteinrichtung 10 aufgegeben. In der Auswerteinrichtung 10 repräsentiert das Verstellsignal VS den Mittelwert der Fadenzugkraft. Die Auswerteinrichtung 10 liefert eine Auswertung des aktuellen Ausgangssignals Z, welches die aktuell gemessene Fadenzugkraft repräsentiert, entsprechend den Grundsätzen, die in der EP-A-207 471 beschrieben sind.

Das bedeutet: In der Auswerteinrichtung 10 ist ein oberer Grenzwert und ein unterer Grenzwert für das Verstellsignal VS eingespeichert (GOVS, GUVS). Wenn das Verstellsignal VS einen dieser Grenzwerte überschreitet, erfolgt ein Alarmsignal. Ferner wird in der Auswerteinrichtung 10 der Differenzwert DU zwischen dem aktuellen Ausgangssignal Z und dem Verstellsignal VS - nachdem beide zuvor in kompatible, vergleichbare Größen gewandelt worden sind - gebildet. Schließlich ist in der Auswerteinrichtung 10 der obere Grenzwert und der untere Grenzwert dieses Differenzsignales DU (GODU, GUDU) gespeichert, und es erfolgt ein Alarmsignal A, wenn das Differenzsignal DU zwischen dem Verstellsignal und dem aktuell gemessenen Ausgangssignal Z einen der Grenzwerte GODU, GUDU überschreitet.

Der obere bzw. untere Grenzwert des Verstellsignals wird in der weiter unten folgenden Beschreibung als Iₘₐₓ bzw. Iₘᵢₙ bezeichnet.

Die Verstelleinrichtung 7 kann z. B. der Antriebsmotor des Friktionsfalschdrallers sein. In diesem Falle erfolgt in der Einrichtung 12 eine Umwandlung des Differenzsignals aus dem Langzeitwert und dem Sollwert in eine Größe, z.B. in eine Frequenz, die die Drehzahl des als Synchronmotor oder als Asynchronmotor ausgelegten Antriebsmotors 7 des Friktionsfalschdrallers bestimmt.

Wenn der Friktionsfalschdraller aus drei parallelen Achsen besteht, die in den Eckpunkten eines gleichseitigen Dreiecks angeordnet sind und auf welchen Scheiben aufgespannt sind, die sich im Zentrum des Dreiecks überlappen, so kann durch die Verstelleinrichtung 7 alternativ oder zusätzlich der Achsabstand verstellt werden, indem z.B. die Achsen auf jeweils einem Exzenter gelagert und die Exzenter in Abhängigkeit von dem Verstellsignal VS entsprechend verdreht werden (vgl. DE-B-21 30 550).

Wenn der Friktionsfalschdraller aus zwei Scheiben besteht, die den Faden zwischen sich einklemmen und von denen eine elastische Scheibe durch eine Andrückeinrichtung gegen die andere Scheibe gedrückt wird (vgl. z.B. EP-A-22 743), so kann durch das Verstellsignal VS die Anpreßkraft verstellt werden. Hierzu wird auch auf die DE-A-33 06 594 verwiesen.

Wenn der Friktionsfalschdraller aus zwei Riemen besteht, die endlos quer zur Fadenachse umlaufen und den Faden zwischen sich einklemmen (z.B. US-A-4,248,038), wobei die Riemenlagerungen mit vorgebbarer Kraft relativ zueinander verstellt werden, so kann durch das Verstellsignal VS diese Kraft verstellt werden.

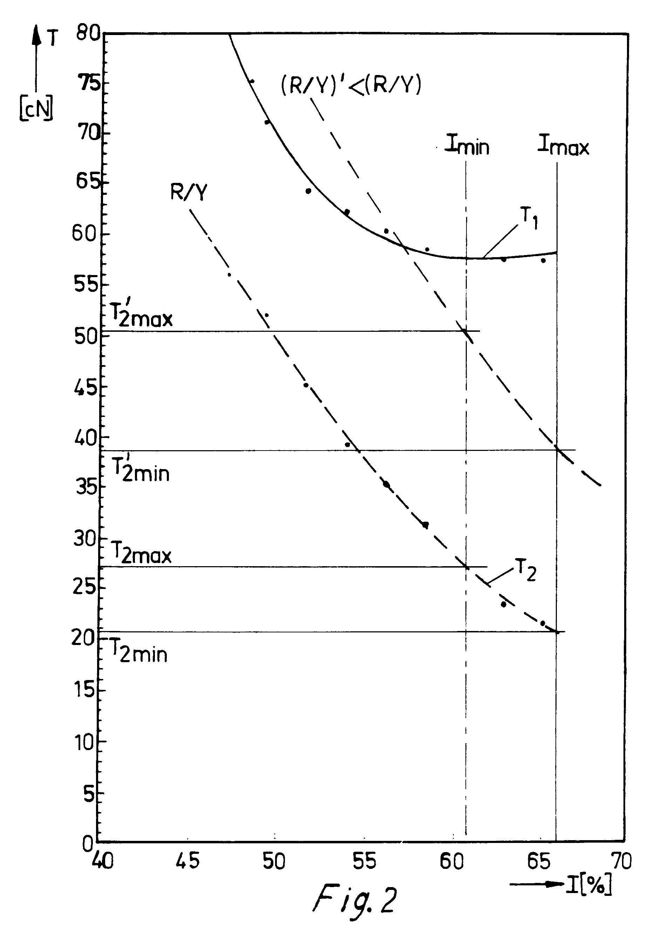

Anhand des Diagramms nach Fig. 2 wird die Vorgehensweise zur Einstellung des Friktionsfalschdrallers und das sich daraus ergebende Regelverfahren beschrieben, und zwar für einen Friktionsfalschdraller, der aus zwei Scheiben besteht, die den Faden zwischen sich einklemmen. Zur Andrückung dient hier ein Magnet und das Verstellsignal wird durch den Magnetstrom I repräsentiert. Wenn im folgenden von dem Strom bzw. Magnetstrom, Mindeststrom, Grenzstrom, Maximalstrom gesprochen wird, so bezieht sich dieser Ausdruck auf die zuvor beschriebene magnetische Andrückeinrichtung. Wenn zur Einstellung der Reibkräfte andere Kraftgeber oder Verstelleinrichtungen benutzt werden, tritt eine entsprechend andere Verstellgröße an die Stelle des Stroms. Der Begriff "Strom" kann also durch den Begriff "Verstellsignal" ersetzt werden.

Es wird zunächst ein Mindeststrom Iₘᵢₙ festgesetzt. Hierzu dient die Kurve T1, welche den Zusammenhang zwischen der Fadenzugkraft T1 vor dem Friktionsfalschdraller und dem Magnetstrom wiedergibt. Es zeigt sich, daß die Fadenspannung T1 einen sehr typischen Verlauf hat. In dem Diagramm nach Fig. 2 ist der Magnetstrom angegeben in Prozent von dem Sättigungsstrom. Die Fadenspannung nimmt nun mit zunehmendem Magnetstrom zunächst sehr stark ab. Es folgt aber sodann ein Bereich, in dem praktisch keine Abhängigkeit zwischen dem Magnetstrom und der Fadenspannung T1 besteht.

Der Mindeststrom Iₘᵢₙ wird in dem Bereich gewählt, in welchem die Fadenspannung T1 von dem Magnetstrom I möglichst nicht abhängig ist. Hierzu wird zunächst ein Grenzstrom I_{grenz} festgelegt. Dieser Strom kennzeichnet die Grenze zwischen den Bereichen der T1-Kurve, in denen einerseits eine starke Abhängigkeit von T1 zu dem Magnetstrom besteht und andererseits keine wesentliche Abhängigkeit von T1 zu dem Magnetstrom besteht. Der Mindestmagnetstrom Iₘᵢₙ wird sodann nahe diesem Grenzstrom gewählt, z. B. 2 bis 10 % größer; ein günstiger Wert liegt bei 5 % größer. Damit ist eine Qualitätsgrenze festgelegt, die die physikalischen Grenzen, wie z. B. das für den Faden erforderliche Drehmoment und die an dem Friktionsfalschdraller erzeugten Reibkräfte berücksichtigt.

Weiter wird nun der Regelbereich festgelegt, der einerseits durch den Mindeststrom Iₘᵢₙ und andererseits durch den maximalen Magnetstrom Iₘₐₓ begrenzt wird. Die Auswahl des maximalen Magnetstroms hängt ab von dem Sättigungsstrom des Magneten. Das ist der Magnetstrom, bei dem Sättigung eintritt, eine weitere Erhöhung der Magnetkraft also nicht mehr zu erzielen ist.

Die Regelung wird aber so betrieben, daß der Sättigungsstrom nicht erreicht wird. Der maximale Magnetstrom Iₘₐₓ wird also jedenfalls niedriger als der Sättigungsstrom gewählt. Für die Festlegung der oberen Stromgrenze Iₘₐₓ sind die folgenden Überlegungen anzustellen: Je größer die Stromgrenze Iₘₐₓ gewählt wird, um so größer ist der Bereich der Fadenzugkräfte, welchen die Regelung ausgleichen kann. Das bedeutet, daß bei großem Regelbereich auch starke Störungen erfaßt und die Fadenzugkraft trotzdem konstant gehalten werden kann. Andererseits bedeutet ein großer Regelbereich aber auch eine Verringerung der Möglichkeit, Störungen und Fehlerquellen zu erkennen. Es ist nun in der EP-A-439 183 ein Verfahren beschrieben, bei dem das Verstellsignal, durch das die Größe und/oder die Komponentenverteilung der Reibkraft des Falschdrallgebers gesteuert wird, zur Qualitätsüberwachung genutzt wird. Dabei wird ein oberer Grenzwert GOVS und ein unterer Grenzwert GUVS für das Verstellsignal vorgegeben und ein Alarmsignal erzeugt oder ein Qualitätssignal ausgegeben, wenn das Verstellsignal den Bereich zwischen dem oberen Grenzwert und dem unteren Grenzwert verläßt. Wenn nun bei dem Verfahren nach dieser Erfindung der obere Grenzwert Iₘₐₓ und der untere Grenzwert Iₘᵢₙ einen großen Regelbereich einschließen, so reagiert die Qualitätsüberwachung nur auf sehr grobe Störungen. Daher bildet die richtige Wahl von Iₘₐₓ einen Kompromiß zwischen einem erwünschten großen Regelbereich auf der einen Seite und einer ausreichend großen Fehlerempfindlichkeit auf der anderen Seite. Als große Hilfe hat sich bei der Suche nach Iₘₐₓ die Möglichkeit gezeigt, in einem Versuch Iₘₐₓ vorzugeben und Fadenmaterial zu erzeugen, bis zum ersten Mal ein Fehlersignal dadurch erfolgt, daß das Verstellsignal, d. h. der Strom den Regelbereich zwischen Iₘₐₓ und Iₘᵢₙ verläßt. Dieses Fadenmaterial wird nun zu einem Strickmuster verarbeitet und die Qualität des Fadenmaterials geprüft. Liegt die Qualität innerhalb der vorgegebenen Toleranz, so kann der maximale Strom Iₘₐₓ weiter erhöht werden, anderenfalls muß er erniedrigt werden. Erwünscht ist die Festlegung von Iₘₐₓ so, daß die durch die Ausregelung der Fadenspannung T2 unterdrückten Störungen nicht zu Fehlern führen, die außerhalb der Toleranzgrenze liegen.

Die obere Stromgrenze Iₘₐₓ ist also eine an Kundenforderungen orientierte Qualitätsgrenze, die je nach Einsatzgebieten unterschiedlich gewählt werden kann.
Der Magnetstrom wird also zwischen dem Mindestmagnetstrom Iₘᵢₙ und dem maximalen Magnetstrom Iₘₐₓ, der kleiner als der Sättigungsstrom ist, verstellt. Nunmehr erfolgt auch eine Festlegung des Regelbereiches für die Fadenspannung T2, indem Störungen der Fadenspannung T2 ausgeregelt werden können. Hierzu wird das Drall/Förder-Verhältnis gewählt durch Wahl des wirksamen Radius R der Scheiben, welcher für die Drallerteilung maßgebend ist und/oder durch Wahl der Fadengeschwindigkeit Y.

Es sind für zwei unterschiedliche Drall/Förder-Verhältnisse (als Parameter) (R/Y) und (R/Y)'die Kurven dargestellt, die den Zusammenhang zwischen der Fadenspannung T und dem Magnetstrom I repräsentieren. Diese Kurven unterscheiden sich durch ihr Niveau und ihre Steilheit. Ein höheres R/Y-Verhältnis bewirkt eine Verschiebung der T2-Kurve nach links, d. h. zu kleineren Stromstärken. Ein niedrigeres R/Y-Verhältnis bewirkt eine Verlagerung der T2-Kurve nach rechts, d. h. zu größeren Stromstärken. Gleichzeitig vergrößert sich aber auch bei größerem R/Y-Verhältnis die Steilheit der Kurven. Bei niedrigerem R/Y-Verhältnis werden daher große Schwankungen der Fadenzugkraft T2 in dem Regelbereich zwischen Iₘᵢₙ und Iₘₐₓ ausgeregelt. Umgekehrt ist der Regelbereich kleiner bei größerem R/Y-Verhältnis. Auch hier geschieht die Auswahl nach den Produktanforderungen.

Das Drall/Förder-Verhältnis (R/Y) wird nun so ausgewählt, daß die Soll-Fadenspannung T2 im Mittel eine bestimmte, gewünschte Höhe hat und dabei durch Veränderung des Magnetstromes I zwischen den beschriebenen Grenzen möglichst auch nur in einem gewünschten Bereich veränderbar ist. Das R/Y-Verhältnis wird also so ausgewählt, daß die damit einstellbare Sollfadenspannung T2ₛₒₗₗ durch Verstellsignale der Reibkraft erzeugbar ist, die im Bereich zwischen GOVS 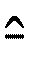 Iₘᵢₙ und GUVS 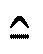 Iₘᵢₙ liegen.

Das dargestellte Diagramm gilt für einen Polyesterfaden 167f30, eine Fadengeschwindigkeit von 850 m/min und ein R/Y-Verhältnis von 1,56. Der Magnetstrom I ist in Prozent des Sättigungsstromes angegeben.

Während des Betriebes wird nun die Fadenspannung T2 in dem zuvor ausgewählten Bereich durch entsprechende Verstellung der Größe und/oder Komponentenverteilung der Reibkraft des Falschdrallgebers am Faden auf einen konstanten Wert im Bereich der Soll-Fadenspannung geregelt. Dabei können Störungen erfaßt werden, die Fadenspannungsänderungen zwischen z. B. T-max und T-min liegen. Diese Störungen rufen Verstellsignale hervor, die zwischen Iₘᵢₙ und Iₘₐₓ liegen. Durch die Erfindung bleibt zwar die Fadenspannung T2 konstant. Sie kann daher nicht mehr zur Signalisierung von Fehlern dienen. Statt dessen werden die Verstellsignale zum Zwecke der Qualitätssicherung laufend erfaßt und vorzugsweise auch aufgezeichnet. Wenn das Verstellsignal den unteren Grenzwert GUVS = Iₘᵢₙ unterschreitet oder den oberen Grenzwert GOVS = Iₘₐₓ überschreitet, greift die Qualitätssicherung ein. Bei Unterschreiten des unteren Grenzwertes werden Fehler signalisiert, deren Ausregelung zu Fehlern in der Fadenspannung T1 und der Drallhöhe, also der Parameter oberhalb des Friktionsfalschdrallers, führen. Bei Überschreiten des oberen Grenzwertes werden zu geringe Fadenspannungen T2 hinter dem Friktionsfalschdraller signalisiert, die insbesondere zu Fehlern der erzeugten Spule führen und sonstige Unregelmäßigkeiten des Prozesses anzeigen.

Es kann ferner die Differenz gebildet werden zwischen der laufend gemessenen Fadenspannung und dem laufend ermittelten Verstellsignal. Dazu ist allerdings zuvor eine Umwandlung der gemessenen Fadenspannung in ein Signal erforderlich, das mit dem Verstellsignal vergleichbar und kompatibel ist. Das Differenzsignal kann ebenfalls laufend überwacht werden. Für das Differenzsignal wird eine obere Grenze GODU und eine untere Grenze GUDU festgesetzt und bei Überschreiten bzw. Unterschreiten der Grenzwerte wird ein Fehler signalisiert. Im einzelnen wird hierzu auf die US-A-4,720,702 sowie die EP-A-0 439 183 Bezug genommen. Wenn ein derartiger Fehler signalisiert wird oder wenn ein oder mehrere Fehler mehrfach und insbesondere mit bestimmter Häufigkeit auftreten oder wenn ein oder mehrere Fehler lange anhaltend auftreten, kann bei Überschreiten einer vorgegebenen Grenze eine Kennzeichnung des Produkts als mindere Qualität oder bei Überschreiten einer Absolutgrenze auch die Unterbrechung der Produktion erfolgen; letzteres insbesondere dann, wenn Häufigkeit, Intensität oder Art des Fehlers darauf schließen lassen, daß die Störungen nicht nur vorübergehender Natur sind.

Gegenüber den bekannten Verfahren läßt sich nach der Erfindung nicht nur eine Qualitätsüberwachung und Qualitätsprüfung, sondern eine weitere wesentliche Qualitätsverbesserung erzielen. Insbesondere wird eine Qualitätsoptimierung durch Regelung der Fadenspannung hinter dem Friktionsfalschdraller erzielt, ohne daß dadurch die für die Qualität maßgebenden Parameter vor dem Friktionsfalschdraller, d. h. Fadenspannung und Drall beeinflußt werden. Hierdurch wird es möglich, die genannten Parameter einzeln und unabhängig voneinander zu optimieren.

## Patentansprüche

1. Verfahren zur Regelung der Fadenzugkraft
des laufenden Fadens hinter dem Friktionsfalschdraller einer Falschzwirnkräuselmaschine,
bei dem die Fadenspannung (T2) hinter dem Friktionsfalschdraller laufend gemessen und dadurch ausgeregelt wird, daß die Fadenspannung über ein Zeitfilter und durch Vergleich mit einer vorgegebenen sollfadenspannung (T2ₛₒₗₗ) in ein Verstellsignal umgewandelt und durch das Verstellsignal die Größe und/oder die Komponentenverteilung der Reibkraft des Falschdrallgebers am Faden gesteuert wird,
mit den kennzeichnenden Merkmalen:
die Abhängigkeit der Fadenspannung (T1) vor dem Friktionsfalschdraller von dem Verstellsignal (VS) wird durch Messung der Fadenspannung (T1) bei Änderung des Verstellsignals (VS) ermittelt und daraus der untere Grenzwert (VSₗᵢₘ; Iₗᵢₘ) des Verstellsignals gewonnen, oberhalb dessen durch Vergrößerung des Verstellsignales (VS) eine qualitätswirksame Beeinflussung des Dralls und/oder der Fadenspannung (T1) in der Drallzone nicht eintritt;
ein Minimalwert (GUVS; Iₘᵢₙ) des Verstellsignales wird festgelegt, das geringfügig, vorzugsweise 1 bis 10 % größer ist als der Grenzwert des Verstellsignales;
ein Maximalwert (GOVS; Iₘₐₓ) des Verstellsignales (VS) wird festgelegt;
die Sollfadenspannung (T2ₛₒₗₗ) wird ausgewählt;
das Drall-/Förderverhältnis (R/Y) wird so gewählt, daß die Sollfadenspannung (T2ₛₒₗₗ) in einem gewünschten Toleranzbereich zwischen einem oberen Grenzwert (T2ₘₐₓ) und und einem unteren Grenzwert (T2ₘᵢₙ) liegt,
wobei das Drall-/Förderverhältnis (R/Y) definiert ist durch den Quotienten zwischen dem wirksamen Radius des Friktionsfalschdrallers und der Fadengeschwindigkeit und dadurch eingestellt wird, daß der Angriffspunkt des Fadens an dem Friktionsfalschdraller oder/und die Fadengeschwindigkeit eingestellt wird
und wobei der obere Grenzwert (T2ₘₐₓ) der Fadenspannung durch Einstellung des Minimalwertes (GUVS; Iₘᵢₙ) und der untere Grenzwert (T2ₘᵢₙ) der Fadenspannung durch Einstellung des Maximalwertes (GOVS; Iₘₐₓ) des Verstellsignals (VS) einstellbar ist.

2. Verfahren nach Anspruch 1,
dadurch gekennzeichnet, daß
ein Fehlersignal erzeugt wird, wenn das Verstellsignal (VS) aus dem durch die Grenzwerte des Verstellsignales
(Iₘᵢₙ/Iₘₐₓ; GOVS/GUVS) definierten Regelbereich herausfällt.

## Claims

1. Method of controlling the tension of the running yarn downstream of the friction false-twisting device of a false twist crimping machine,
whereby the yarn tension (T2) downstream of the friction false-twisting device is continuously measured and corrected in that the yarn tension is converted by means of a time filter and through comparison with a preselected setpoint yarn tension (T2ₛₒₗₗ) into an adjustment signal and the adjustment signal is used to control the magnitude and/or component distribution of the frictional force of the false-twist generator on the yarn,
having the characterizing features:
the dependence of the yarn tension (T1) upstream of the friction false-twisting device upon the adjustment signal (VS) is determined by measuring the yarn tension (T1) during variation of the adjustment signal (VS) and is utilized to obtain the lower limit value (VSₗᵢₘ; Iₗᵢₘ) of the adjustment signal, above which an increase in the adjustment signal (VS) does not lead to a quality-affecting influencing of the twist and/or the yarn tension (T1) in the twist zone;
a minimum value (GUVS; Iₘᵢₙ) of the adjustment signal is fixed which is slightly greater, preferably 1 to 10% greater, than the limit value of the adjustment signal;
a maximum value (GOVS; Iₘₐₓ) of the adjustment signal (VS) is fixed;
the setpoint yarn tension (T2ₛₒₗₗ) is selected;
the twist-feed ratio (R/Y) is selected in such a way that the setpoint yarn tension (T2ₛₒₗₗ) lies within a desired tolerance range between an upper limit value (T2ₘₐₓ) and a lower limit value (T2ₘᵢₙ),
the twist/feed ratio being defined by the ratio between the effective radius of the friction false-twisting device and the yarn speed and being set by adjusting the point of application of the yarn against the friction false-twisting device and/or the yarn speed
and the upper limit value (T2ₘₐₓ) of the yarn tension being adjustable by setting the minimum value (GUVS; Iₘᵢₙ) and the lower limit value (T2ₘᵢₙ) of the yarn tension being adjustable by setting the maximum value (GOVS; Iₘₐₓ) of the adjustment signal (VS).

2. Method according to claim 1,
characterized in that
an error signal is produced if the adjustment signal (VS) falls outside of the control range defined by the limit values of the adjustment signal (Iₘᵢₙ/Iₘₐₓ; GOVS/GUVS).

## Revendications

1. Procédé de régulation de la force de traction du fil en mouvement en aval du générateur de fausse torsion par friction d'une machine de frisage par fausse torsion, suivant lequel on mesure la tension de fil (T2) en permanence en aval du générateur de fausse torsion par friction et la régule par le fait qu'on transforme la tension de fil en passant par un filtre de temporisation et par comparaison avec une tension de fil de consigne prédéterminée (T2 _{cons}) en un signal de commande et on fait agir ce signal de commande sur la valeur et/ou la répartition des composantes de la force de friction du générateur de fausse torsion sur le fil, caractérisé par le fait qu'on détermine la dépendance de la tension de fil (T1) en amont du générateur de fausse torsion par friction par rapport au signal de commande (VS) par mesure de la tension de fil (T1) en cas de variation du signal de commande (VS), et produit la valeur limite inférieure (VSₗᵢₘ; Iₗᵢₘ) du signal de commande, valeur au-dessus de laquelle une augmentation du signal de commande (VS) n'a pas d'action de qualité sur la torsion et/ou la tension de fil (T1) dans la zone de torsion; on fixe une valeur minimale (GUVS; Iₘᵢₙ) du signal de commande, qui est légèrement, de préférence de 1 à 10%, supérieure à la valeur limite du signal de commande; on fixe une valeur minimale (GOVS; Iₘₐₓ) du signal de commande (VS); on sélectionne la tension de fil de consigne (T2_{cons}); on choisit le rapport torsion/transport (R/Y) de manière que la tension de fil de consigne (T2_{cons}) soit située dans une plage de tolérances désirées entre une valeur limite supérieure (T2ₘₐₓ) et une valeur limite inférieure (T2ₘᵢₙ), le rapport torsion/transport (R/Y) étant défini par le quotient du rayon utile du générateur de fausse torsion par friction et la vitesse de fil et étant réglé par le fait que le point d'attaque du fil sur le générateur de fausse torsion et/ou la vitesse de fil est réglé, et la valeur limite supérieure (T2ₘₐₓ) de la tension de fil étant réglable par réglage de la valeur minimale (GUVS; Iₘᵢₙ) du signal de commande et la valeur limite inférieure (T2ₘᵢₙ) de la tension de fil étant réglable par réglage de la valeur maximale (GOVS; Iₘₐₓ) du signal de commande (VS).

2. Procédé suivant la revendication 1, caractérisé par le fait qu'on produit un signal de défaut lorsque le signal de commande (VS) sort de la plage de régulation définie par les valeurs limites du signal de commande (Iₘᵢₙ/Iₘₐₓ; GOVS/GUVS).
